# EUROPEAN PATENT SPECIFICATION

(11) **EP 1 786 381 B1**
(45) Date of publication and mention of the grant of the patent: **03.12.2014**
(21) Application number: 05773767.8
(22) Date of filing: 26.08.2005
(51) Int. Cl.: A61K 6/00

(54) **ANTI-BACTERIAL COMPOUNDS**
ANTIBAKTERIELLE VERBINDUNGEN
COMPOSES ANTIBACTERIENS

(30) Priority: 06.09.2004 GB 0419693
(43) Date of publication of application: 23.05.2007
(73) Proprietor: Beiersdorf AG, 20253 Hamburg (DE)
(72) Inventor: DERRER, Samuel, CH-8117 Fällanden (CH); NATSCH, Andreas, CH-8707 Uetikon (CH); TRAUPE, Bernd, 24568 Kaltenkirchen (DE); STANG, Melanie, CH-4313 Möhlin (CH)
(74) Representative: Hartmann, Jost
(86) International application number: PCT/CH2005/000501
(87) International publication number: WO 2006/026875

(56) References cited:
- EP-A- 0 400 610
- EP-A- 0 432 039
- EP-A- 0 887 336
- WO-A-01/59067
- WO-A2-01/59067
- DE-A1- 2 241 862
- JP-A- 2002 316 968
- US-A- 2 068 905
- US-A- 4 204 879
- US-A- 4 767 768
- US-A- 5 879 469
- US-A1- 2001 033 854
- DATABASE REGISTRY [Online] 16 November 1984 (1984-11-16), XP002352682 retrieved from STN Database accession no. RN64219-54-1
- DATABASE REGISTRY [Online] 16 November 1984 (1984-11-16), XP002352683 retrieved from STM Database accession no. RN77927-88-9
- DATABASE REGISTRY [Online] 16 November 1984 (1984-11-16), XP002352684 retrieved from STN Database accession no. RN86240-15-5
- DATABASE REGISTRY [Online] 9 June 1985 (1985-06-09), XP002352685 retrieved from STN Database accession no. RN96655-13-9
- DATABASE REGISTRY [Online] 25 March 1998 (1998-03-25), XP002352686 retrieved from STN Database accession no. RN203120-80-3
- DATABASE REGISTRY [Online] 8 May 2002 (2002-05-08), XP002352687 retrieved from STN Database accession no. RN412271-04-6
- DATABASE REGISTRY [Online] 27 July 1999 (1999-07-27), XP002352688 retrieved from STN Database accession no. RN229026-30-6
- DATABASE REGISTRY [Online] 17 March 2004 (2004-03-17), XP002352689 retrieved from STN Database accession no. RN663923-52-2
- DATABASE CA [Online] CHEMICAL ABSTRACTS SERVICE, COLUMBUS, OHIO, US; YE, WEIZHEN ET AL: "Extractant for separation of rare-earth metals" XP002352690 retrieved from STN Database accession no. 1995:677337 & CN 1 084 574 CN (SHANGHAI ORGANIC CHEMISTRY INSTITUTE, CHINESE ACADEMY OF SCIENCES, PEO) 8 May 2002 (2002-05-08)
- DATABASE CA [Online] CHEMICAL ABSTRACTS SERVICE, COLUMBUS, OHIO, US; BIALER, MEIR ET AL: "Pharmacokinetic and pharmacodynamic analysis of (E)-2-ene valproyl derivatives of glycine and valproyl derivatives of nipecotic acid" XP002352691 retrieved from STN Database accession no. 1996:610664 & BIOPHARMACEUTICS & DRUG DISPOSITION , 17(7), 565-575 CODEN: BDDID8; ISSN: 0142-2782, 1996,
- HADAD S, BIALER M: "Pharmacokinetic analysis and antiepileptic acitivity of two new isomers of N-valproyl glycinamide" BIOPHARMACEUTICS AND DRUG DISPOSITION, vol. 18, no. 7, 1997, pages 557-566, XP009056582
- GRANNEMAN G R, WNAG S I, MACHINIST J M, KESTERSON J W: "Aspects of the metabolism of valproic-acid" XENOBIOTICA, vol. 14, no. 5, 1984, pages 375-388, XP009056581
- CHEMICAL ABSTRACTS, vol. 110 Columbus, Ohio, US; abstract no.: 134696, MATSUDA, MASANORI ET AL: "Study of branching alkoxyacetic acids by carbon-13 NMR",

## Description

The present invention relates to antibacterial compounds, their use in consumer products and methods of making the same.

As used herein, the term "anti-bacterial" used in connection with a compound of the present invention is intended to refer to a compound that displays bacteriostatic or bactericidal properties, or both, depending on the condition to be prevented or treated and the concentration of compound or compounds employed.

Conditions caused by bacteria, in particular bacteria present on the human skin, include body malodour such as axillary malodour and foot odour, and acne. Body malodour is formed when certain compounds contained in fresh perspiration, which are essentially odourless, are catabolised by bacteria such as Staphylococci and Corynebacteria , both of which genera belong to the class of gram-positive Eubacteriaceae. Regarding foot malodour, a major cause of this condition is the presence of Brevibacteria under high humidity and low aeration conditions. Acne is another skin manifestation attributed to a bacterial origin. The microorganism thought to be responsible is Propionibacterium acnes.

Anti-bacterial agents are used in consumer products to prevent or treat these conditions.

Anti-bacterial compositions generally incorporate active agents that reduce such bacterial flora on the skin or in the household. However, a problem with many known anti-bacterial agents is that they may affect the entire microbial flora and not just the targeted micro-organism.

Many anti-bacterial agents are useful in treating one or more of the aforementioned conditions. Triclosan is an anti-bacterial agent used in many products including household and personal care products. However', being a chlorinated product, its use is questioned by consumer protection organisations. Further, it has especially high activity against low odour-forming Staphylococci bacteria, and as such may create favourable conditions in which the more problematic Corynebacteria may thrive.

There have been many disclosures in the art of perfume ingredients that have anti-bacterial properties. One such natural fragrance compound is Farnesol. However, the problem with using perfume ingredients as anti-bacterial agents is that, to obtain anti-bacterial effects, they must generally be employed at higher levels than one would customarily wish to use in fragrance applications. Further, even if such materials could be used to achieve an anti-bacterial effect at low levels suitable for perfumers, their volatility is often so high that they will only be effective for a short period of time before they evaporate and leave the skin.

Non-perfume compounds have been employed as anti-bacterial agents in household and consumer products including personal care products and products to be applied to the skin. The use of monolaurin-glycerol and other glycerol esters or glycerol mono-ethers have been described in the art.

Applicant has now found a new class of compounds that are odourless and that have a reasonable residence time on the skin of a user, thereby to exert a desirable anti-bacterial effect.

Therefore the invention provides in one of its aspects a compound according to the formula I 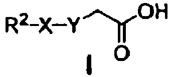 wherein Y is O, wherein X is selected from CO and CH2, and wherein R2 is a branched saturated or unsaturated hydrocarbon moiety selected from C7-C15 according to formula II 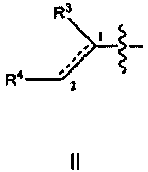 wherein the bond between C1 and C2 (C1 and C2 being the carbon atoms in position 1 or 2 as indicated in formula II) is a single bond, or preferably a double bond, and R3 and R4 are independently selected from methyl, ethyl, propyl, butyl, pentyl, hexyl, heptyl and octyl, nonyl, decyl, undecyl and dodecyl, and a monocyclic aralkyl residue optionally substituted with a C1, C2, C3, C4 or C5 alkyl for use as an anti-bacterial medicament in the form of a product to be applied to human skin.

By "singly-branched" is meant a hydrocarbon moiety with a main carbon and one carbon chain branch.

C7-C15 specifically include C7 , C8, C9, C10, C11, C12, C13, C14 and C15.

Branched hydrocarbons moieties of R2 are meant to include aliphatic residues, alicyclic residues optionally substituted with C1 to C5 alkyl, and monocyclic aralkyl residues optionally substituted with C1 to C5 alkyl. However, compounds according to formula I wherein R2 is aliphatic are preferred.

Compounds according to formula I may have the following combinations of X and Y residues: X = CO and Y = O, X = CH2 and Y = O.

For their excellent antibacterial activity, compounds according to formula I, wherein Y is 0, or wherein X is CO are preferred; even more preferred are compounds wherein Y is 0 and X is CO.

For the aliphatic R3 and R4, this allows for the following combinations as indicated in table 1 and 2. Each double column indicates R2 in the first row, and the different possibilities of R3 and R4 in the following rows of each double column.

**Table 1**

| **R²=C₇** | | **R²=C₈** | | **R²=C₉** | | **R²=C₁₀** | | **R²=C₁₁** | |
|---|---|---|---|---|---|---|---|---|---|
| **R³** | **R⁴** | **R³** | **R⁴** | **R³** | **R⁴** | **R³** | **R⁴** | **R³** | **R⁴** |
| methyl | butyl | methyl | pentyl | methyl | hexyl | methyl | heptyl | methyl | octyl |
| ethyl | propyl | ethyl | butyl | ethyl | pentyl | ethyl | hexyl | ethyl | heptyl |
| propyl | ethyl | propyl | propyl | propyl | butyl | propyl | pentyl | propyl | hexyl |
| butyl | methyl | butyl | ethyl | butyl | propyl | butyl | butyl | butyl | pentyl |
| | | pentyl | methyl | pentyl | ethyl | pentyl | propyl | pentyl | butyl |
| | | | | hexyl | methyl | hexyl | ethyl | hexyl | propyl |
| | | | | | | heptyl | methyl | heptyl | ethyl |
| | | | | | | | | octyl | methyl |

**Table 2**

| **R²=C₁₂** | | **R²=C₁₃** | | **R³=C₁₄** | | **R²=C₁₅** | |
|---|---|---|---|---|---|---|---|
| **R³** | **R⁴** | **R³** | **R⁴** | **R³** | **R⁴** | **R³** | **R⁴** |
| methyl | nonyl | methyl | decyl | methyl | undecyl | methyl | dodecyl |
| ethyl | octyl | ethyl | nonyl | ethyl | decyl | ethyl | undecyl |
| propyl | heptyl | propyl | octyl | propyl | nonyl | propyl | decyl |
| butyl | hexyl | butyl | heptyl | butyl | octyl | butyl | nonyl |
| pentyl | pentyl | pentyl | hexyl | pentyl | heptyl | pentyl | octyl |
| hexyl | butyl | hexyl | pentyl | hexyl | hexyl | hexyl | heptyl |
| heptyl | propyl | heptyl | butyl | heptyl | pentyl | heptyl | hexyl |
| octyl | ethyl | octyl | propyl | octyl | butyl | octyl | pentyl |
| nonyl | methyl | nonyl | ethyl | nonyl | propyl | nonyl | butyl |
| | | decyl | methyl | decyl | ethyl | decyl | propyl |
| | | | | undecyl | methyl | undecyl | ethyl |
| | | | | | | dodecyl | methyl |

Even more preferred are compounds according to formula I with R2 a hydrocarbon moiety according to formula II and wherein R3 and R4 are selected from the following combinations: R3= ethyl and R4 = propyl, R3 = butyl and R4 = methyl, R3 = butyl and R4 = pentyl, R3 = hexyl and R4 = propyl, R3 = hexyl and R4 = heptyl, R3 = octyl and R4 = pentyl.

A preferred anti-bacterial effect is an effect against human skin bacteria, including typical malodour-forming bacteria present in the human axilla (including Corynebacterium and Staphylococcus), and typical malodour forming bacteria present on the human foot (including Brevibacteria).

A compound according to the present invention exerts good antimicrobial activity when used in consumer products, products applied in the household or on the human body, particularly when applied to the skin or scalp, for example as part of personal care product, as a part of a shampoo composition for application to the scalp, or as part of a composition for application to the feet of a person in need of treatment. Compounds of the present invention exert a bacteriostatic effect that is comparable to or better than that of known bacteriostatic agents, for example Triclosan, glycerol esters, such as monolaurin-glycerol, known glycerol ethers, or known fragrance oils. In tests, the bacteriostatic Minimal Inhibitory Concentration (MIC) of the compounds may range from 0.0004% to greater than 0.5% (weight/volume) depending on the microorganism treated. These values, including the values for many of the common bacteria, and in particular the Corynebacteria, compare favourably with known glycerol mono-esters or mono-ethers, Triclosan, and a known antibacterial fragrance compound, Farnesol. Further details as to how the MIC test data are generated are provided in Example 14 below.

In addition, some compounds of the present invention exert a bactericidal effect. In tests, the anti-bacterial Minimal Bactericidal Concentration (MBC) of the compounds ranges from 0.002% to greater than 0.5% (weight/volume) depending on the micro-organism treated. Further details as to how the MBC test data are generated are provided in Example 15 below.

Compounds of the present invention may be formed as follows. Compounds of formula I with Y=O and X=CH2 (I-a), and compounds of formula I with Y=O and X=CO (I-b) are most conveniently prepared in one step from bromo-acetic acid and the corresponding alcohol or carboxylic acid under basic conditions (compare I-a and I-b in scheme 1, in example 1).

After the reaction, the compounds of formula I (I-a and I-b) are distilled, purified by chromatography or re-crystallisation or isolated without purification.

Further details of the preparative methods are disclosed in the examples herein below.

The present invention provides in another of its aspects the use of at least one compound as hereinabove described as an active agent in compositions such as consumer products for the prevention or treatment of bacterial conditions, and/or for the elimination or suppression of malodour, particularly conditions or malodour resulting from the presence of malodour-forming bacteria on the human skin (for example, axillary malodour, foot malodour, and acne)

Further, the invention provides a non-therapeutic use of a compound according to formula I as defined above as an anti-bacterial or malodour counteracting agent in products selected from consumer products, household products, cosmetic and personal care products, products for use on the human body, products applied to the human skin, and perfumed consumer goods.

Consumer products include household products, cosmetic and personal care products, products for use on the human body, products applied to the human skin, and perfumed consumer goods.

Cosmetic and personal care products, in particular deodorant and antiperspirant cosmetic or personal care products, include soaps, sticks, roll-ons, sprays, pump-sprays, aerosols, deodorant soaps, soap bars, powders, solutions, gels, creams, balms and lotions, eau de Cologne, and eau de toilet, deodorant soaps, shampoos, bath salt, salves, lotions, creams, and ointments.

Perfumed consumer goods include sprays, detergents and solid fragranced products such as powders, soaps, detergent powders, tissues, fabrics, room deodorizers, room deodorizing gels, and candles.

The amount of a compound employed in such a composition will depend upon the nature of the product and the condition to be treated. However, in the case of products that are applied to and left on the skin, it is preferred that a compound or mixtures of compounds be present in said compositions in an amount of about 0.1 to about 2.0% by weight, preferably about 0.1 to 1% by weight of the total composition.

If a compound or a mixture of compounds is to be used in a composition intended to be applied to, and subsequently rinsed off, the skin or scalp, e.g. a shampoo composition, then it is preferred to use the compound or mixture of compounds in higher amount, for example from about 1.0 to 5.0% by weight of the total composition.

Depending on the nature of the consumer product, compounds of the present invention may also be combined with art-recognised quantities of other excipients commonly employed in these products; useful selections may be found in « CTFA Cosmetic Ingredient Handbook », J. M. Nikitakis (ed.), 1st ed., The Cosmetic, Toiletry and Fragrance Association, Inc., Washington, 1988, which is hereby incorporated by reference.

In general, excipients may for example, include colorants, fragrances, solvents, surfactants, colorants, opacifiers, buffers, antioxidants, vitamins, emulsifiers, UV absorbers, silicones and the like. All products can also be buffered to the desired pH using commonly-available excipients in a known manner.

Excipients in deodorant cologne may comprise ethanol and fragrance. Fragrance may be present from 1 to 10% and the ethanol may be present to make up the mass to 100% by weight. A typical ethanol-free deodorant stick may comprise polyols, such as propylene glycol; derivatives thereof, such as propylene-glycol-3-myristyl ether (for example Witconol® APM) ; water; a surfactant such as sodium stearate; and a fragrance. The polyol may be present to the extent of 30 to 40% by weight; the derivatives of the polyol likewise may be present to the extent of 30 to 40% by weight; water may be present to to the extent of 10 to 20% by weight; the surfactant may be present to the extent of 5 to 10% by weight; and the fragrance may be present to the extent of as mentioned above.

A typical antiperspirant stick may comprise excipients such as Ethylene Glycol Monostearate (e.g. from 5 to 10%); Shea butter (e.g. from 3 to 5%); Caprylic/Capric Triglyceride such as Neobee® 1053 (PVO International) (e.g. from about 12 to 15%); phytosterols such as General® 122 (Henkel) (e.g. from about 3 to 7%); Dimethicone (DC 345) (e.g. from 30 to 40%); aluminium sesquichlorohydrate (for example from about 15 to 20%); and a fragrance, for example from 1 to 10%.

An antiperspirant aerosol may contain as excipients ethanol, typically to the extent of 10 to 15%; zirconium aluminium tetrachlorohydrate, for example from 3 to 5%; Bentone 38, for example from 1 to 2%; fragrance in an amount aforementioned; and a hydrocarbon propellant, such as S-31 , for example up to 100%.

An antiperspirant pump composition may contain as excipients aluminium sesquichlorohydrate, for example from 15 to 25%; water, for example from 50 to 60%; an octylphenol ethoxylate non-ionic surfactant such as Triton X-102 (Union carbide), for example from 1 to 3%; dimethyl Isosorbide (ICI), for example from 15 to 25 %; and a fragrance in an amount as aforementioned.

All percentages mentioned above are in wt %.

In all the above mentioned compositions, the compound or mixtures thereof may of use as the sole active agent, or it may be used in conjunction with other active agents such as Triclosan (CAS 3380-34-5) or other commercially-available antibacterial or anti-fungal agents, or with known fragrance oils having anti-bacterial or anti-fungal properties.

There now follows a series of non-limiting examples that serve to illustrate the invention. Formulae where Y = NH are illustrative and do not fall under the claimed invention.

### Example 1

### Overview synthesis of compounds according to formula I

Compounds of formula I are most conveniently prepared by the transformations shown in Scheme 1. Compounds of formula I with Y=O and X=CH2 (I-a), and compounds of formula I with Y=O and X=CO (I-b) are accessible in one step from bromo acetic acid and the corresponding alcohol or carboxylic acid under basic conditions (Scheme Ia, b). In the case of compounds of formula I with Y=NH and X=CO (I-c), commercial glycine ethyl ester hydrochloride is used as starting material for the condensation and the ester protecting group is subsequently removed under alkaline conditions (Scheme Ic). The acids of formulae I-a to I-c are distilled, purified by chromatography or recyrstallisation or isolated without purification.

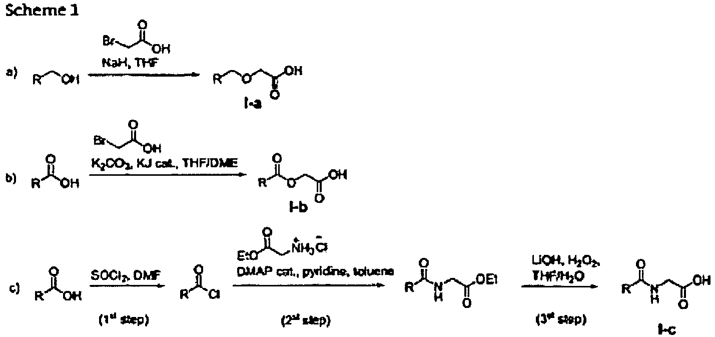

### Example 2

### Procedure for the synthesis of compounds I-a

To a stirred solution of the alcohol (0.1 mol) in THF (125 ml) is added in portions sodium hydride (0.22 mol) at room temperature. The grey suspension is stirred for 30 min. and then cooled to O °C. Bromo acetic acid (0.1 mol) is added in portions without exceeding 5 0C. After the mixture is heated to reflux for 6-8 h the white suspension is allowed to cool to room temperature, concentrated and the residue taken up in water and fert-butyl methyl ether. The organic layer is washed with aqueous saturated sodium hydrogencarbonate, water and brine. The dried (Na2SO4) ether phase is concentrated In vacuo to afford the desired Compounds of formula I with Y=O and X=CH2 (I-a) as a colourless oil in a yield of 15-90% depending on the chain length of the alcohol.

### Example 3

### Procedure for the synthesis of compounds I-b

Potassium carbonate (0 55 mol) and potassium iodide (0025 mol) is suspended m 1,2-dimethoxyethane (600 ml) and to this stirred mixture is added the carboxylic acid at room temperature After stirring for 30 mm the mixture is cooled to 0°C and bromo acetic acid is added in portions within 15 mm Then the white slurry is heated to reflux for 4-8 h before it is allowed to cool to room temperature The reaction mixture is quenched with aqueous hydrochloric acid (10%) and extracted with tert -butyl methyl ether The combined organic phases are washed with water and brine, dried over sodium sulfate and the solvent evaporated Depending on the nature of the carboxylic acid the desired compounds of formula I with Y=O and X=CO (I-b) are obtained in a yield of 30-75% as a colourless oil

### Example 4

### General procedure (C) for the synthesis of compounds I-c

1st step: To the carboxylic acid (0.39 mol) and N,B'-dimethylformamide (0.5 g) is added under stirring thionyl chloride (0.48 mol) within 15 min. The mixture is stirred for 4 h when TLC shows the conversion to be completed. The excess thionyl chloride is distilled off and yielded quantitative the carboxylic acid chloride as a colourless oil
2st step A stirred mixture of glycine ethylester hydrochloride (57 mmol), N,N-dimethylamino pyridine (5 7 mmol) and pyridine (200 mmol) in toluene (250 ml) is cooled to 0 °C and the above acid chloride is added withm 10 mm After 2 h stirring m an ice bath the mixture is allowed to warm to room temperature and quenched with aqueous hydrochloric acid (5%) The aqueous phase is extracted with tert-butyl methyl ether, the combined organic layers are washed with water and brine and dried over Na2SO4 Removing of the solvent gives the glycine ethylester amide in a yield of 65-100% as a white solid
3st step The above ester (2 9 mmol) is dissolved m tetrahydrofuran/water (60 ml, 3 1) and cooled to 0°C by an ice bath Aqueous hydrogen peroxide (30%, 116 mmol) and lithium hydroxide (5 8 mmol) is added and the ice bath removed The mixture is stirred for 15 h after which it is quenched with aqueous sodium sulfite solution (saturated) under stirring for 10 mm Then the solvent is removed, the residue taken up in aqueous hydrochloric acid (10%) and extracted with ethyl acetate The organic phase is washed with water and brine, dried over sodium sulfate and concentrated m vacuo The desired compounds of formula I with Y=NH and X=CO (I-c) are obtained in a yield of 80-95% as a white solid

### Examples 5 - 8

In the following, spectroscopic data is presented of compounds of formula I with Y=O and X=CH2 (I-a), which are prepared according to example 2

### Example 5:

### [3-(4-tert.-Bulyl-phenyl)-2-methyl-propoxy]-acetic acid

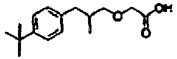

H-NMR (200 MHz, CDCl3, coupling constants in Hz): 0.95 (3H, d, /8, CH3); 1.31 (9H, s, 3 x CH3); 2.03-2.15 (1H, m, CH); 2.44 (1H, dd, /13.6, 7.8CH2); 2.71 (1H, dd, /13.6, 6.3 CH2); 3.36-3.47 (2H, m, CH2); 4.10 (1H, s, CH2); 7.09 (2H, d, /8.3, 2 x ArH); 7.30 (2H, d, /8.3, 2 x ArH).
IR (vmax, cm-1, ATR): 3250 brw, 2960m, 1725s, 1133s, 850w, 800w.
MS [m/z (EI)]: 264 (M+, 22%), 249 (35), 138 (32), 173 (84), 147 (31), 131 (100).

### Example 6:

### (2-Ethyl-hexyloxy)-acetic acid

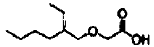

H-NMR (400 MHz, CDCl3, coupling constants in Hz): 0.85-0.94 (6H, m, 2 x CH3); 1.24- 1.48 (8H, m, 4 x CH2); 1.52-1.61 (1 H, m, CH); 3.45 (2H, d, /6.1, CH2); 4.12 (2H, s, CH2); 1.00 (1H, br s, COOH).
IR (vmax, cm-1, ATR): 3150 brw, 2959m, 2928m, 1726s, 1243m, 1130s, 933w.
MS [m/z (EI)]: 188 (M+, <1%), 129 (8), 111 (10), 83 (17), 70 (37), 69 (35), 57 (100), 43 (40), 41 (40), 29 (19).

### Example 7:

### (2-Butyl-octyloxy)-acetic acid

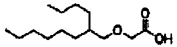

H-NMR (400 MHz, CDCl3, coupling constants in Hz): 0.84-0.92 (6H, m, 2 x CH3); 1.19- 1.38 (16H, m, 8 x CH2); 1.57-1.63 (1H, m, CH); 3.45 (2H, d, /5.8, CH2); 4.10 (2H, s, CH2); 9.00-10.50 (1H, br, COOH).
IR (vmax, cm-1, ATR): 3200brw, 2925s, 2857m, 1726s, 1243m, 1130s, 692w.
MS [m/z (EI)]: 244 (M+, <1%), 185 (5), 111 (27), 97 (32), 85 (32), 83 (32), 71 (45), 69 (44), 57 (100), 43 (71), 41 (41).

### Example 8:

### (2-Hexyl-decyloxy)-acetic acid

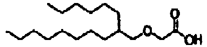

H-NMR (400 MHz1 CDCl3, coupling constants in Hz): 0.85-0.91 (6H, m, 2 x CH3); 1.21- 1.39 (24H, m, 12 x CH2); 1.57-1.66 (1 H, m, CH); 3.45 (2H, d, /5.8, CH2); 4.09 (2H, s, CH2); 9.00-10.50 (1 H, br, COOH).
IR (vmax, cm-1, ATR): 3100brw, 2923s, 2855m, 1727m, 1243w, 1131m, 723w.
MS [m/z (EI)]: 300 (M+, 9%), 241 (27), 139 (24), 125 (35), 111 (80), 97 (90), 83 (89), 71 (92), 57 (100), 55 (90), 43 (94).

### Examples 9 - 11:

In the following, spectroscopic data is presented of compounds of formula I with Y=O and X=CO (I-b), which are prepared according to example 3.

### Example 9:

### 2-Ethyl-hexanoic acid carboxymethyl ester

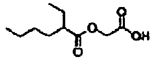

H-NMR (400 MHz, CDCl3, coupling constants in Hz): 0.85-0.97 (6H, m, 2 x CH3); 1.22- 1.37 (4H, m, 2 x CH2); 1.44-1.75 (4H, m, 2 x CH2); 2.34-2.43 (1 H, m, CH); 4.67 (2H, S, CH2).
IR (vmax, cm-1, ATR): 3200brw, 2935w, 2875w, 1732s, 1141s, 1102m, 799w.
MS [m/z (EI)]: 203 (M-H+, 1%), 174 (11), 146 (70), 127 (29), 98 (53), 118 (18), 70 (100), 57 (59), 55 (37).

### Example 10:

### 2-Butyl-octanoic acid carboxymethyl ester

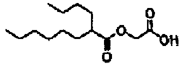

H-NMR (400 MHz, CDCl3, coupling constants in Hz): 0.82-0.94 (6H, m, 2 x CH3); 1.19- 1.38 (12H, m, 6 X CH2); 1.42-1.54 (2H, m, CH2); 1.58-1.72 (2H, m, CH2); 2.40-2.48 (1 H, m, CH); 4.66 (2H, s, CH2).
IR (vmax. cm-1, ATR): 3100brw, 2929m, 28759w, 1734s, 114Os1 1113m, 728w.
MS [m/z (EI)]: 259 (M-H+, <1%), 202 (4), 183 (11), 174 (31), 126 (41), 98 (100), 70 (100), 55 (29).

### Example 11:

### 2-Hexyl-decanoic acid carboxymethyl ester

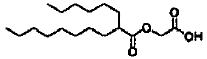

H-NMR (400 MHz, CDCl3, coupling constants in Hz): 0.82-0.93 (6H, m, 2 x CH3); 1.18-1.36 (20H, m, 10 X CH2); 1.41-1.54 (2H, m, CH2); 1.58-1.72 (2H, m, CH2); 2.39-2.49 (1 H, m, CH); 4.66 (2H, s, CH2).
IR (vmax, cm-1, ATR): 3100brw, 2925s, 2856m, 1735s, 1459w, 1150s, 724w.
MS [m/z (EI)]: 315 (M-H+, <1%), 239 (8), 202 (8), 154 (24), 126 (54), 98 (100), 84 (23), 55 (28).

### Examples 12 -13:

In the following is presented spectroscopic data of compounds of formula I with Y=NH and X=CO (I-c), which are prepared according to example 4.

### Example 12:

### (2-Butyl-octanoylamino)-acetic acid

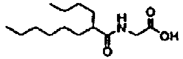

H-NMR (400 MHz, CDCl3, coupling constants in Hz): 0.84-0.91 (6H, m, 2 x CH3); 1.19- 1.34 (12H, m, 6 x CH2); 1.37-1.51 (2H, m, CH2); 1.54-1.67 (2H, m, CH2); 2.05-2.15 (1H, m, CH); 4.08-4.11 (2H, m, CH2); 5.98-6.03 (1 H, br t, NH).
IR (vmax, cm-1, ATR): 3283m, 2926m, 2852m, 1734m, 1711m, 1644s, 1544s, 1244m, 1234s, 679m.
MS [m/z (EI)]: 258 (M-H+, 8%), 201 (51), 173 (74), 130 (100), 126 (29), 55 (38).

### Example 13:

### (2-Hexyl-decanoylamino)-acetic acid

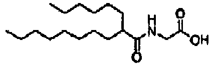

H-NMR (400 MHz, CDCl3, coupling constants in Hz): 0.83-0.91 (6H, m, 2 x CH3); 1.19-1.33 (2OH, m, 10 x CH2); 1.37-1.49 (2H, m, CH2); 1.54-1.65 (2H, m, CH2); 2.05-2.15 (1H, m, CH); 4.09 (2H, d, /5.3, CH2); 6.04 (1 H, br t, /5.0, NH).
IR (vmax, cm-1, ATR): 3293w, 2922m, 2852m, 1736m, 1643s, 1542m, 1232m, 931w.
MS [m/z (EI)]: 314 (M-H+, 5%), 229 (41), 130 (100), 130 (100), 126 (35), 117 (31), 76 (36), 57 (34), 55 (52), 43 (44).

### Example 14:

### Bacteriostatic activity of the compounds of the present invention

The bacteriostatic effects of the compounds of the present invention are compared with those of known anti-bacterial agents against human skin bacteria. Typical bacteria (Corynebacterium and Staphylococcus strains) isolated from the human axilla and commercially available strains are used. The results are shown below in Table 1.

The different axilla bacteria are isolated from the human axilla according to techniques known in the art, in particular according to standard microbiological practice. They are taxonomically identified by cell morphology, gram-reaction and biochemical tests included in the Api coryne test kit (BioMerieux, France) . Strain Staphylococcus epidermidis Ax25 is identified by fatty acid methyl ester analysis (FAME; German type strain collection DSMZ, Germany). Escherichia coli DSM 682, Staphylococcus aureus DSM 799, and Corynebacterium xerosis DSM 20170 are obtained from the German-type strain collection. The strains are maintained on Tryptic soy broth plates, this standard medium being supplemented with 5 g per Litre of Polyoxyethylene Sorbitan Monooleate (Tween™ 80). Plates are incubated at 36° C for a period of 48 hours. The bacteria are then swabbed from the plates and suspended in 4 ml of Müller-Hinton broth supplemented with 100 mg of Tween™ 80 per Litre (MH-Tween) and incubated again at 36°C for 16 hours. Following incubation the bacterial suspensions are diluted in MH-Tween to obtain a final cell density of 107 colony forming units per ml. These diluted suspensions of the different test organisms are distributed to different columns of a microtiter plate, 100 µl per well. The test compounds are dissolved in dimethylsulfoxide (DMSO) at various test concentrations and 2.5 µl of these different DMSO solutions are added to the different wells of the test plates. The plates are covered with plastic films and incubated for 24 h at 36°C with shaking at 250 rpm. The turbidity developing in the microtiter platesi then examined after 24 h to determine microbial growth. The minimal concentration of test compounds inhibiting the growth of an organism by at least 80% is determined as the minimal inhibitory concentration (MIC).

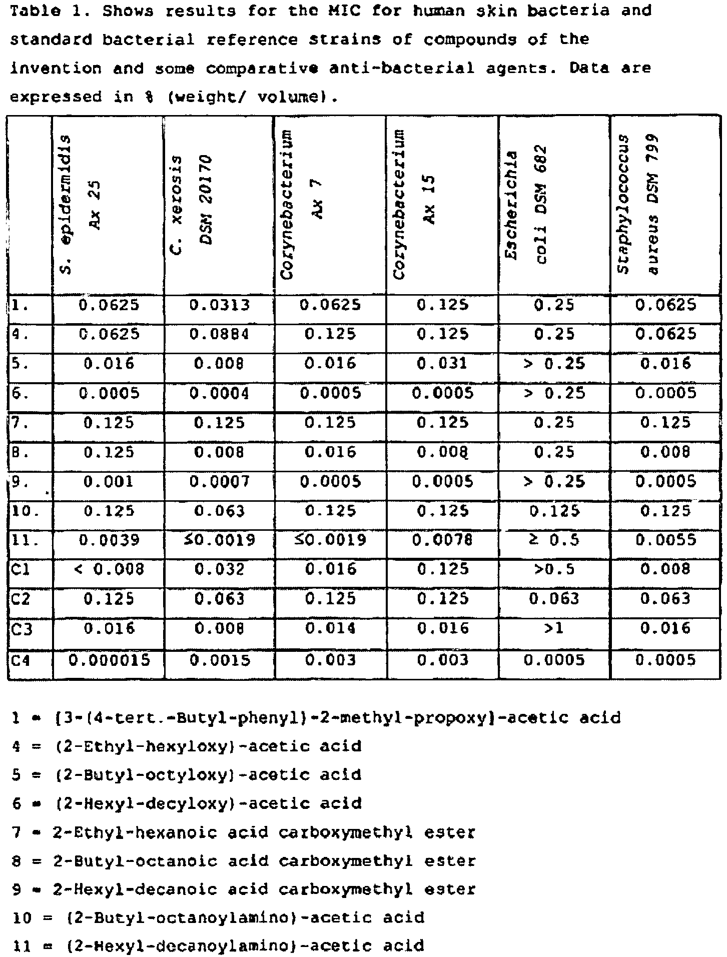

C1 = Dodecanoic acid 2, 3-d1Hydroxy-propyl ester: (1-mon-lauroyl-glycerol),
C2 = a commercial glycerol mono-ether: 3- (2-Ethyl-hexyloxy) -propane-1, 2-diol,
C3 = Farnesol,
C4 = Triclosan

Ax 25 is identified as Staphylococcus epidermidis by FAME analysis. Ax 7 is identified as Corynebacterium group G and Ax 15 as Corynebacterium jeikeium with the Api Coryne test kit. The latter two strains are isolated from the axilla of human volunteers and are able to generate axilla malodour when incubated in vitro with human axilla secretions.

One can see from Table 1 that for all strains studied, most compounds of the present invention are better performing than the mono-ether (C2) and are comparable and better than the mono-ester (CI) and Farnesol (C3), which commonly are used in perfumes, personal care products and perfumed consumer goods. The compounds are particularly active against the odour forming skin bacteria and some of the compounds are more active then the chlorinated antibacterial compound Triclosan when tested against the malodour forming bacteria Ax7 and Ax15.

### Example 15:

### Bactericidal activity of the compounds of the present invention

The bactericidal effects of the compounds of the present invention are further tested. The results are shown below in Table 2.

The bacteria are grown, harvested and diluted under the same conditions as described in the example above and added to microtiter plates (100 µl per well), each well containing 2.5 µl of the different DMSO solutions as described above. After 60 min incubation, 0.5 µl of the bacterial culture from each well (corresponding to 5 x 103 cfu in the control treatment) is transferred to a fresh microtiter plate which contained 100 µl per well of fresh medium. The plates are covered with plastic films and incubated for 24 h at 36°C with shaking at 250 rpm. The turbidity developing in the microtiter plates is then examined after 24 h to determine microbial growth. The minimal concentration of test compounds which completely killed the bacterial inoculum is determined as the minimal bactericidal concentration (MBC).

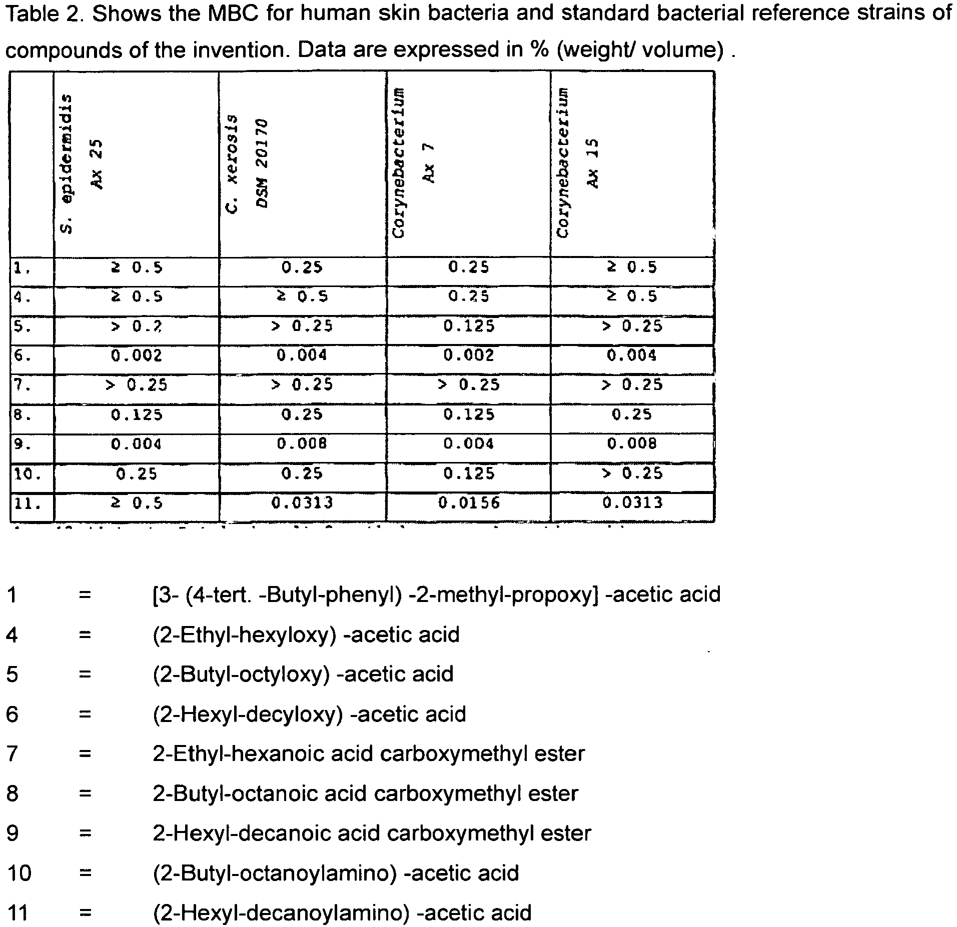

One can see from Table 2 that, for all strains studied, some compounds of the present invention have not only a bacteriostatic activity, but also the inherent potential to completely kill an inoculum of 3 x 103 cfu within 60' contact time at a very low test concentration. The compounds are particularly active against the odor forming skin bacteria.

### Example 16

### Aerosol Spray

| | I | II | III |
|---|---|---|---|
| Octyldodecanol | 0,50 | 0,50 | 0,50 |
| Propylene Glycol | 1,00 | 1,00 | 1,00 |
| 2-Hexyl-decanoic acid carboxymethyl ester | 0,50 | - | - |
| (2-Butyl-octyloxy)-acetic acid | - | 0,50 | - |
| (2-Hexyl-decyloxy)-acetic acid | - | - | 0,50 |
| Perfume | q.s. | q.s. | q.s. |
| Ethanol | ad 100,00 | ad 100,00 | ad 100,00 |

The mixed liquid phase is filled with a mixture of propane-butane (2:7) in proportion of 39:61 in a spray can

### Example 17

### Roll-on Gel

| | I | II | III |
|---|---|---|---|
| Ethanol | 50,00 | 50,00 | 50,00 |
| Polyoxyethylen-(20)- sorbitanmonolaurat | 2,00 | 2,00 | 2,00 |
| Hydroxyethylcellulose | 0,50 | 0,50 | 0,50 |
| 2-Hexyl-decanoic acid carboxymethyl ester | 0,50 | - | - |
| (2-Butyl-octyloxyl-acetic acid | - | 0,50 | - |
| (2-Hexyl-decyloxy)-acetic acid | - | - | 0,50 |
| Aluminium Chlorohydrat | 10,00 | 10,00 | 10,00 |
| Perfume | q.s. | q.s. | q.s. |
| Water | ad 100,00 | ad 100,00 | ad 100,00 |

### Example 18

### Antiperspirant Stick:

| | I | II | III |
|---|---|---|---|
| Stearylalkohol | 25,00 | 20,00 | 20,00 |
| PEG-40 Hydrogenated Castor oil | 2,00 | 3,00 | 3,00 |
| Cyclomethicone | ad 100 | ad 100 | ad 100 |
| 2-Hexyl-decanoic acid carboxymethyl ester | 0,50 | - | - |
| (2-Butyl-octyloxy)-acetic acid | - | 0,50 | - |
| (2-Hexyl-dacyloxy)-acetic acid | - | - | 0,50 |
| Aluminium Chlorohydrat, Powder | 20,00 | 25,00 | 25,00 |
| Perfume | q.s. | q.s. | q.s. |

## Claims

1. A compound, or a mixture of compounds, selected from the group of substances according to formula I 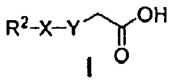 wherein Y is O, wherein X is selected from CO and CH₂,
and wherein R² is a branched saturated or unsaturated hydrocarbon moiety selected from C₇-C₁₅ according to formula II, 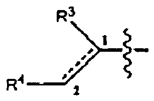 wherein the bond between C1 and C2 is a single bond, or a double bond, and R³ and R⁴ are independently selected from methyl, ethyl, propyl, butyl, pentyl, hexyl, heptyl and octyl, nonyl, decyl, undecyl and dodecyl, and a monocyclic aralkyl residue optionally substituted with a C₁, C₂, C₃, C₄ or C₅ alkyl, for use as an anti-bacterial medicament in the form of a product to be applied to human skin.

2. A compound, or a mixture of compounds for use according to claim 1, wherein R² is aliphatic.

3. A compound, or a mixture of compounds for use according to claim 1 or 2, wherein X is CO.

4. A compound, or a mixture of compounds for use according to claim 1, 2 or 3, wherein R² is saturated.

5. A compound, or a mixture of compounds for use according to claim 4 selected from the group consisting of
a compound with R³= ethyl and R⁴= propyl,
a compound with R³ = butyl and R⁴ = methyl,
a compound with R³= butyl and R⁴= pentyl,
a compound with R³= hexyl and R⁴ = propyl,
a compound with R³ = hexyl and R⁴ = heptyl,
a compound with R³ = octyl and R⁴ = pentyl.

6. A compound or a mixture of compounds for use according to claim 1 selected from the group consisting of 3-(4-tert.-Butyl-phenyl)-2-methyl-propoxy]-acetic acid; (2-Ethyl-hexyloxy)-acetic acid; (2-Butyl-octyloxy)-acetic acid; (2-Hexyl-decyloxy)-acetic acid; 2-Ethyl-hexanoic acid carboxymethyl ester; 2-Butyl-octanoic acid carboxymethyl ester; 2-Hexyl-decanoic acid carboxymethyl ester.

7. A composition comprising a compound, or a mixture of compounds according to claim 1 to 6, wherein the compound or mixture of compounds is present in an amount of from 0.1 to 5.0% by weight, for use as an anti-bacterial medicament in the form of a product to be applied to human skin.

8. A composition for use according to claim 7 wherein the compound or mixture of compounds is present in an amount of from 0.1 to 1% by weight.

9. A compound or a mixture of compounds according to claim 1 to 6 for the use as a medicament against acne.

10. Non-therapeutic use of a compound as defined in any of the claims 1 to 6 as an anti-bacterial or malodour counteracting agent in products selected from consumer products, household products, cosmetic and personal care products, products for use on the human body, products applied to the human skin, and perfumed consumer goods.

11. Use according to claim 9 in a composition wherein the compound or mixture of compounds is present in an amount of from 0.1 to 5.0% by weight.

12. Use according to claim 9 in a composition wherein the compound or mixture of compounds is present in an amount of from 0.1 to 1.0% by weight.

## Patentansprüche

1. Verbindung oder Gemisch von Verbindungen aus der Gruppe von Substanzen gemaß Formel I 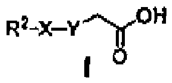 worin Y für O steht, X aus CO und CH₂ ausgewählt ist
und worin R² für eine verzweigte gesättigte oder ungesättigte Kohlenwasserstoffgruppierung steht, die aus C₇-C₁₅ gemäß Formel II 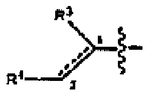 ausgewählt ist, wobei die Bindung zwischen C1 und C2 eine Einfachbindung oder eine Doppelbindung ist und R³ und R⁴ unabhängig aus Methyl, Ethyl, Propyl, Butyl, Pentyl, Hexyl, Heptyl und Octyl, Nonyl, Decyl, Undecyl und Dodecyl und einem monocyclischen Aralkylrest, der gegebenenfalls durch ein C₁-, C₂-, C₃-, C₄- oder C₅-Alkyl substituiert ist, ausgewählt sind, zur Verwendung als antibakterielles Medikament in Form eines auf menschliche Haut aufzubringenden Produkts.

2. Verbindung oder Gemisch von Verbindungen zur Verwendung nach Anspruch 1, wobei R² aliphatisch ist

3. Verbindung oder Gemisch von Verbindungen zur Verwendung nach Anspruch 1 oder 2, wobei X für CO steht.

4. Verbindung oder Gemisch von Verbindungen zur Verwendung nach Anspruch 1, 2 oder 3, wobei R² gesättigt ist.

5. Verbindung oder Gemisch von Verbindungen zur Verwendung nach Anspruch 4, ausgewählt aus der Gruppe bestehend aus
einer Verbindung mit R³ = Ethyl und R⁴ = Propyl,
einer Verbindung mit R³ = Butyl und R⁴ = Methyl,
einer Verbindung mit R³ = Butyl und R⁴ = Pentyl,
einer Verbindung mit R³= Hexyl und R⁴ = Propyl,
einer Verbindung mit R³ = Hexyl und R⁴ = Heptyl,
einer Verbindung mit R³ = Octyl und R⁴ = Pentyl.

6. Verbindung oder Gemisch von Verbindungen zur Verwendung nach Anspruch 1, ausgewählt aus der Gruppe bestehend aus 3-(4-tert.-Butylphenyl)-2-methyl-propoxy]essigsäure; (2-Ethylhexyloxy)essigsäure: (2-Butyloctyloxy)essigsäure; (2-Hexyldecyloxy)essigsäure; 2-Ethylhexansäurecarboxymethylester; 2-Butyloctansäurecarboxymethylester; 2-Hexyldecansäurecarboxymethylester.

7. Zusammensetzung, umfassend eine Verbindung oder ein Gemisch von Verbindungen nach Anspruch 1 bis 6, wobei die Verbindung bzw. das Gemisch von Verbindungen in einer Menge von 0,1 bis 5,0 Gew,-% vorliegt, zur Verwendung als antibakterielles Medikament in Form eines auf menschliche Haut aufzubringenden Produkts.

8. Zusammensetzung zur Verwendung nach Anspruch 7, wobei die Verbindung bzw. das Gemisch von Verbindungen in einer Menge von 0,1 bis 1 Gew.-% vorliegt.

9. Verbindung oder Gemisch von Verbindungen nach Anspruch 1 bis 6 zur Verwendung als Medikament gegen Akne.

10. Nichttherapeutische Verwendung einer Verbindung gemäß einem der Ansprüche 1 bis 6 als antibakterielles oder schlechten Geruch bekämpfendes Mittel in Produkten, die aus Konsumprodukten, Haushaltsprodukten, Kosmetik- und Körperpflegeprodukten, Produkten zur Verwendung auf dem menschlichen Körper, auf die menschliche Haut aufgetragenen Produkten und parfümierten Konsumgütern ausgewählt sind.

11. Verwendung nach Anspruch 9 in einer Zusammensetzung, in der die Verbindung bzw. das Gemisch von Verbindungen in einer Menge von 0,1 bis 5,0 Gew.-% vorliegt.

12. Verwendung nach Anspruch 9 in einer Zusammensetzung, in der die Verbindung bzw. das Gemisch von Verbindungen in einer Menge von 0,1 bis 1,0 Gew.-% vorliegt.

## Revendications

1. Composé ou mélange de composés, choisi dans le groupe de substances répondant à la formule I 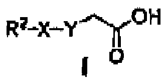 où Y représente O, où X est choisi parmi CO et CN₂,
et où R² représente une entité hydrocarbonée saturée ou insaturée ramifiée choisie parmi C₇-C₁₅ répondant à la formule II, 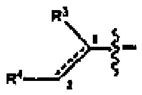 où la liaison entre C1 et C2 représente une liaison simple, ou une liaison double, et chacun des radicaux R³ et R⁴ est indépendamment choisi parmi les groupements méthyle, éthyle, propyle, butyle, pentyle, hexyle, heptyle et octyle, nonyle, décyle, undécyle et dodécyle, et un résidu arylalkyle monocyclique éventuellement substitué par un groupement alkyle en C₁, C₂, C₃, C₄ ou C₅, pour emploi en tant que médicament antibactérien sous la forme d'un produit à appliquer à la peau humaine.

2. Composé ou mélange de composés pour emploi conforme à la revendication 1, où R² représente un groupement aliphatique.

3. Composé ou mélange de composés pour emploi conforme à la revendication 1 ou 2, où X représente un groupement CO.

4. Composé ou mélange de composés pour emploi conforme à la revendication 1, 2 ou 3, où R² représente un groupement saturé.

5. Composé ou mélange de composés pour emploi conforme à la revendication 4,
choisi dans le groupe constitué par
un composé où R³ = éthyle et R⁴ = propyle,
un composé où R³ = butyle et R⁴ = méthyle,
un composé où R³= butyle et R⁴ = pentyle,
un composé où R³ = hexyle et R⁴ = propyle,
un composé où R³ = hexyle et R⁴ = heptyle,
un composé où R³ = octyle et R⁴ = pentyle.

6. Composé ou mélange de composés pour emploi conforme à la revendication 1 choisi dans le groupe constitué par les suivants : Acide 3-(4-tert.-butyl-phényl)-2-méthyl-propoxy]-acétique ; Acide (2-éthyl-hexyloxy)-acétique ; Acide (2-butyl-octyloxy)-acétique ; Acide (2-hexyl-décyloxy)-acétique ; 2-Éthyl-hexanoate de carboxyméthyle ; 2-Butyl-octanoate de carboxyméthyle ; 2-Hexyl-décanoate de carboxyméthyle.

7. Composition comprenant un composé ou un mélange de composés conforme à la revendication 1 à 6, où le composé ou le mélange de composés est présent à une teneur comprise entre 0,1 et 5,0 % en masse, pour emploi en tant que médicament antibactérien sous la forme d'un produit à appliquer à la peau humaine.

8. Composition pour emploi conforme à la revendication 7, où le composé ou le mélange de composés est présent à une teneur comprise entre 0,1 et 1 % en masse,

9. Composé ou mélange de composés conforme à la revendication 1 à 6, pour emploi en tant que médicament contre l'acné.

10. Emploi non thérapeutique d'un composé conforme à l'une quelconque des revendications 1 à 6 en tant qu'agent antibactérien ou anti-mauvaises odeurs dans des produits choisis parmi les produits de consommation, les produits ménagers, les produits de soins personnels et cosmétiques, les produits pour emploi sur le corps humain, les produits appliqués à la peau humaine, et les produits de consommation parfumés.

11. Emploi conforme à la revendication 9 dans une composition où le composé ou le mélange de composés est présent à une teneur comprise entre 0,1 et 5,0 % en masse.

12. Emploi conforme à la revendication 9 dans une composition où le composé ou le mélange de composés est présent à une teneur comprise entre 0,1 et 1,0 % en masse.
